# FASCICULE DE BREVET EUROPEEN

(11) **EP 1 857 046 B1**
(45) Date de publication et mention de la délivrance du brevet: **17.06.2009**
(21) Numéro de dépôt: 07290609.2
(22) Date de dépôt: 15.05.2007
(51) Int. Cl.: A61B 5/00, A61B 5/103, G01W 1/00

(54) **Dispositif de mesure du climat sous-vestial et vêtements associés**
Vorrichtung zur Messung des Klimas unter einer Jacke und entsprechendes Kleidungsstück
Device for measurement of the microclimate and associated clothes

(30) Priorité: 16.05.2006 FR 0604312
(43) Date de publication de la demande: 21.11.2007
(73) Titulaire: Etat Français représenté par le Délégué Général pour l'Armement, 94114 Arcueil Cedex (FR)
(72) Inventeur: Savourey Gustave, 38330 Biviers (FR); Besnard Yves, 38400 Saint Martin d'Heres (FR); Launay Jean-Claude, 38000 Grenoble (FR)

(56) Documents cités:
- EP-A1- 1 486 763
- EP-A2- 0 846 440
- WO-A-20/04034045
- FR-A1- 2 292 970
- JP-A- 8 131 406
- KR-A- 2002 095 294

## Description

La présente invention concerne notamment le domaine de la mesure de température de parties du corps et a plus particulièrement pour objet un dispositif de mesure du climat sous-vestial d'un individu du type comportant au moins un capteur de température et un capteur d'humidité.
Les interventions opérationnelles et d'une façon générale toute activité humaine en environnement climatique contraignant peut induire des pathologies graves qui nécessitent une prévention. La contrainte thermique résulte des effets de l'environnement climatique lui-même, mais aussi de l'activité physique (dépense énergétique) et des contraintes thermiques imposées par les équipements, contraintes dépendant entre autres des caractéristiques thermiques de ceux-ci notamment de leur résistance thermique et évaporatoire. Ainsi, lors du port de vêtements et/ou de tenues de protection, les échanges thermiques ne se font plus de la peau du sujet vers l'ambiance mais de la peau vers le vêtement puis du vêtement vers l'ambiance, voire l'inverse. Une telle situation peut conduire à une hyperthermie avec un risque d'accident grave si l'activité n'est pas arrêtée à temps.

La demande de brevet US20020013538 tente de résoudre ce problème en proposant un dispositif de contrôle des signes de santé d'un individu, tels que la pression sanguine ou la température, comportant, dans ce dernier cas, au moins un capteur de température disposé à proximité de la peau de l'individu et des moyens de transmission des signaux caractéristiques des valeurs mesurées par le capteur, ainsi que des moyens de réception et de traitement de signaux auxquels sont associés des moyens de climatisation et/ou des moyens d'alerte de l'atteinte ou du dépassement d'un seuil pouvant entraîner une pathologie, la transmission et la réception pouvant être réalisées par un réseau sans fil, par exemple de type RF.

Toutefois, cette demande de brevet, d'une part, ne décrit pas la structure des capteurs de température utilisés et, d'autre part, la mesure de la température de la peau n'est pas suffisante pour déterminer un risque clinique.

On connaît aussi l'abrégé du brevet JP2005126877 qui décrit un dispositif de climatisation portable par une personne auquel sont associés des capteurs de température et d'humidité, d'une part, au niveau de la peau de la personne et, d'autre part, au niveau de la surface externe du vêtement porté par la personne. Un tel dispositif présente un inconvénient majeur : en effet, la mesure de l'humidité absolue de la peau est toujours proche de la pression saturante de vapeur d'eau à la température cutanée mesurée du fait, même au repos, de la perspiration insensible, cette mesure étant donc nullement représentative de l'état physiologique de la personne qui l'utilise.

On connaît par ailleurs l'abrégé du brevet JP5010826 qui décrit une structure comportant des capteurs de température ou d'humidité en divers points de la surface de la peau et comportant un support flexible rectangulaire sur lequel sont disposés des électrodes sur lesquelles reposent lesdits capteurs.

Un tel dispositif comporte le même inconvénient que celui cité dans le cadre du brevet JP2005126877, à savoir la mesure de l'humidité absolue de la peau n'est nullement représentative de l'état physiologique de la personne qui l'utilise.

Enfin, on connaît le brevet KR20020095294 qui décrit un dispositif de mesure de température et d'humidité dans le but d'améliorer le confort des vêtements.

Un tel dispositif comporte également les mêmes inconvénients que les brevets précités.

Le but de l'invention est de résoudre les inconvénients précités en proposant un dispositif apte à mesurer des caractéristiques représentatives de l'état physiologique d'un individu.

La solution apportée est selon une première caractéristique, un dispositif de mesure de la température et de l'humidité de l'air situé entre la peau d'un individu et la paroi d'un vêtement ou d'un autre équipement en vis-à-vis de la peau de l'individu, possèdant une ossature comportant, latéralement selon son épaisseur e :
- une première partie constituée par un premier support ajouré apte à être disposé contre la peau de l'individu,
- une seconde partie constituée par ladite paroi ou par un second support ajouré apte à être disposé contre cette paroi,
- des moyens de liaison ajourés aptes à solidariser la première partie à la seconde partie et constitués soit par une entretoise soit par plusieurs entretoises aptes à permettre une circulation par convexion naturelle de d'air situé entre la peau et ladite paroi au travers du volume délimité par l'ossature,
- au moins un capteur de température et un capteur d'humidité disposés au niveau du plan médian P1 de l'ossature.

Le volume délimité par l'ossature est celui délimité par l'enveloppe comportant :
- la plus grande section de la première partie,
- la plus grande section de la seconde partie, voire à nouveau la plus grande section de la première partie lorsque la seconde partie consiste en la paroi du vêtement ou de l'équipement,
- la surface virtuelle réunissant le périmètre de la plus grande section de la première partie à celui de la plus grande section de la seconde partie telle que définie précédemment.

Ainsi dans le cas où les surfaces principales des première et deuxième parties ont la même dimension ℓ x L et que l'ossature a une épaisseur e, le volume de l'ossature est égal à V=ℓ x L x e

Un tel dispositif, placé entre la peau et la paroi interne du vêtement d'un individu permet, de par le ou les ajourements de la première partie, une évaporation et un échange thermique quasi normale de la peau qui leur fait face avec l'air situé entre la peau et ladite paroi. Il en est de même pour la seconde partie.

Par moyens de liaison ajourés, il faut entendre que leur section transversale est, dans un plan donné transversal, inférieure, à celle, correspondante, du volume de l'ossature, permettant ainsi une circulation de l'air notamment perpendiculairement audit plan transversal entre lesdites première et seconde parties et au-delà.

Ce ou ces ajourements des moyens de liaison permettent à l'air situé entre la peau et ladite paroi de pouvoir traverser l'ossature et donc d'éviter tout confinement de l'air au niveau du volume délimité par l'ossature, un tel confinement conduisant à des mesures erronées de température et d'humidité.

Par ailleurs, ladite paroi peut-être la paroi interne d'un vêtement ou d'un autre équipement tel par exemple un sac de couchage, un drap, une couverture etc....

Selon une caractéristique particulière, la section transversale des moyens de liaison est, dans un plan donné transversal P2, inférieure, à celle, correspondante, du volume de l'ossature, leur rapport étant, au maximum égal à 1/2.

Selon une caractéristique particulière, au moins l'une de la ou des entretoises est constitué par un tige.

Selon une caractéristique particulière, chacune des dites première et seconde parties est constituée par un cadre ou par une grille.

Selon une caractéristique favorisant la stabilité du dispositif, chacune des dites parties est constituée par une plaque et, préférablement, chacune des plaques comporte deux faces principales et quatre faces secondaires de plus petite taille, le capteur étant disposé entre deux faces principales appartenant chacune à l'une des plaques.

Selon une autre caractéristique, chacune des dites deux parties comporte deux faces principales et l'une au moins des deux dimensions principales de ces parties est supérieure à l'épaisseur e de l'ossature.

Selon une autre caractéristique, un dispositif selon l'invention comporte des moyens de transmission de signaux.

L'invention concerne aussi un dispositif de mesure du climat sous-vestial d'un individu, caractérisé en ce qu'il comporte au moins un et préférablement au moins quatre dispositifs selon l'invention aptes à générer des signaux représentatifs de la température et de l'humidité de l'air entourant chacun des capteurs disposés au sein de ces dispositifs.

Selon une caractéristique particulière, un tel dispositif comporte en outre des moyens d'acquisition et de stockage des signaux provenant de chacun des dispositifs.

L'invention concerne aussi un vêtement ou un autre équipement comportant une face interne destinée à être positionnée en regard de la peau de l'individu qui le porte et une face externe opposée, et caractérisé en ce qu'il comporte au moins un dispositif selon l'invention fixé sur sa face interne.

Elle concerne aussi un vêtement, au moins pour le haut du corps, tel un T-shirt, une chemise, un pull, une veste, une combinaison ... et comportant des manches, caractérisé en ce qu'il comporte, fixé sur sa paroi interne destinée à être en vis-à-vis avec la peau de l'individu qui le porte, au moins un dispositif selon l'invention au niveau de l'une au moins des manche, au niveau du torse et au niveau de la taille. Dans le cas d'une combinaison, elle comporte, d'une manière préférentielle, également au moins un dispositif selon l'invention au niveau d'au moins l'une des jambes.

Dans le cas où le vêtement comporte une capuche, elle comporte, d'une manière préférentielle, également au moins un dispositif selon l'invention sur sa paroi interne.

D'autres avantages et caractéristiques apparaîtront dans la description de plusieurs modes de réalisation de l'invention au regard des figures annexées parmi lesquelles :
- Les figures 1 a et 1b, montrent un schéma d'un dispositif de mesure de température et d'humidité selon un premier mode de réalisation de l'invention,
- la figure 2 présente un schéma d'un dispositif de mesure de température et d'humidité selon un second mode de réalisation de l'invention,
- la figure 3 expose un schéma d'un dispositif de mesure de température et d'humidité selon une variante de réalisation du second mode de réalisation de l'invention,
- la figure 4 montre un schéma d'un dispositif de mesure de température et d'humidité selon une autre variante de réalisation du second mode de réalisation de l'invention,
- la figure 5 présente un schéma d'un dispositif de mesure du climat sous-vestial d'un individu,
- La figure 6 présente un exemple d'évolution de la pression partielle moyenne de vapeur d'eau du climat sous-vestial mesurée avec un dispositif de mesure du climat sous-vestial selon l'invention.

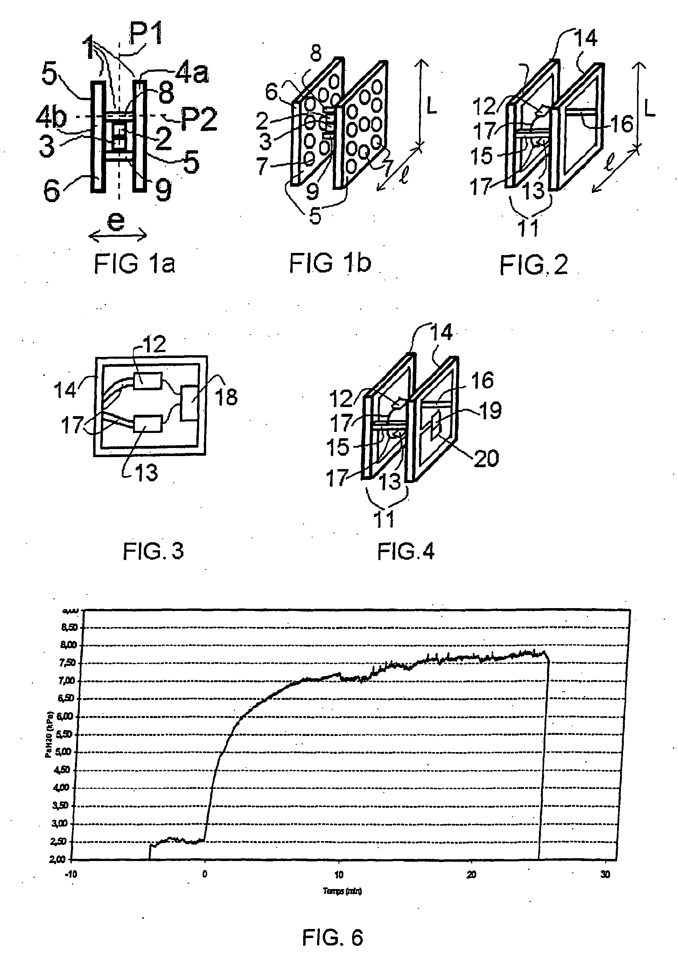

Comme montré sur les figures 1 a et 1b, un dispositif de mesure de température et d'humidité selon ce premier mode de réalisation de l'invention comporte une ossature 1 comprenant une première partie 4a constituée par un premier support ajouré, une seconde partie 4b, identique à la première et constituée par un second support ajouré et des moyens de liaison ajourés 8,9 aptes à solidariser la première partie à la seconde partie et un capteur de température 2 et un capteur d'humidité 3 disposé au niveau du plan médian P1 de l'ossature 1.

Chacune de ces première et seconde parties 4a, 4b a la forme d'une plaque comportant deux faces principales 5 et quatre faces secondaires 6 ainsi que des ouvertures 7 débouchant au niveau de chacune des faces principales 5. Ces première et seconde parties 4a, 4b de l'ossature 1 sont solidarisées entre-elles par deux tiges 8 et 9 servant d'entretoise et sur lesquelles sont fixés de façon connue lesdits capteurs 2 et 3.

Afin de favoriser la stabilité du capteur lors de son utilisation pour mesurer la température et l'humidité à proximité d'un objet, la largeur ℓ et la longueur L des faces principales 5 desdites parties principales 4a, 4b sont supérieures à l'épaisseur e de l'ossature 1. En l'occurrence, l'épaisseur e de l'ossature 1 est, dans ce mode de réalisation d'environ 10mm, la largeur ℓ et la longueur L de chacune des faces principales 5 étant de l'ordre de 20mm. Ainsi, le volume délimité par l'ossature est égal à ℓ × L x e soit 4 cm³.

Pour mesurer la température et l'humidité à proximité d'un individu, il suffit de déposer l'une des faces principales libres du dispositif contre la peau de cet individu, l'autre face principale libre étant contre la paroi interne d'un vêtement porté par l'individu, par exemple fixée par collage à ladite paroi. Ainsi, ce n'est ni la température de la peau ni son humidité qui sont mesurées mais une température et une humidité mesurées à une distance d'environ 5mm de la peau, ces valeurs étant représentatives du climat sous-vestial local.

La figure 2 montre un dispositif selon un deuxième mode de réalisation de l'invention comportant une ossature 11 sur laquelle sont fixés un capteur de température 12 et un capteur d'humidité 13.

L'ossature 11 est constituée de deux parties 14 ajourées constituées chacune par un cadre de forme carrée et solidarisées entre-elles par une première et une seconde tiges 15, 16, les capteurs de température 12 et un capteur d'humidité 13 étant fixés à l'extrémité de petites tiges 17 fixées à leur autre extrémité à la première tige 15, les capteurs se retrouvant ainsi dans le plan médian de l'ossature.

Afin de favoriser la stabilité du capteur lors de son utilisation pour mesurer la température et l'humidité à proximité d'un objet, la largeur ℓ et la longueur L du cadre sont supérieures à l'épaisseur e de l'ossature. En l'occurrence, l'épaisseur de l'ossature est, dans ce mode de réalisation d'environ 5 mm, la largeur ℓ et la longueur L de chacune des cadres 14 étant de l'ordre de 10 mm. Ainsi, le volume délimité par l'ossature est égal à ℓ x L x e soit 0,5 cm³.

La figure 3 montre un dispositif selon le deuxième mode de réalisation de l'invention mais comportant en outre des moyens 18 de transmission HF des signaux générés par les capteurs de température et d'humidité 12 et 13, les moyens 18 de transmission HF étant fixés à la seconde tige 16.

La figure 4 montre un dispositif selon le deuxième mode de réalisation de l'invention mais comportant en outre un capteur de fréquence cardiaque 19 fixé à l'un des cadres 14 et destiné à être positionné contre la peau du thorax par exemple. Un autre capteur 20, par exemple un capteur de saturation en oxygène et/ou de pouls et/ou de pression artérielle, peut aussi être disposé à proximité du capteur de fréquence cardiaque 19, par exemple fixé à ce dernier comme montré sur cette figure.

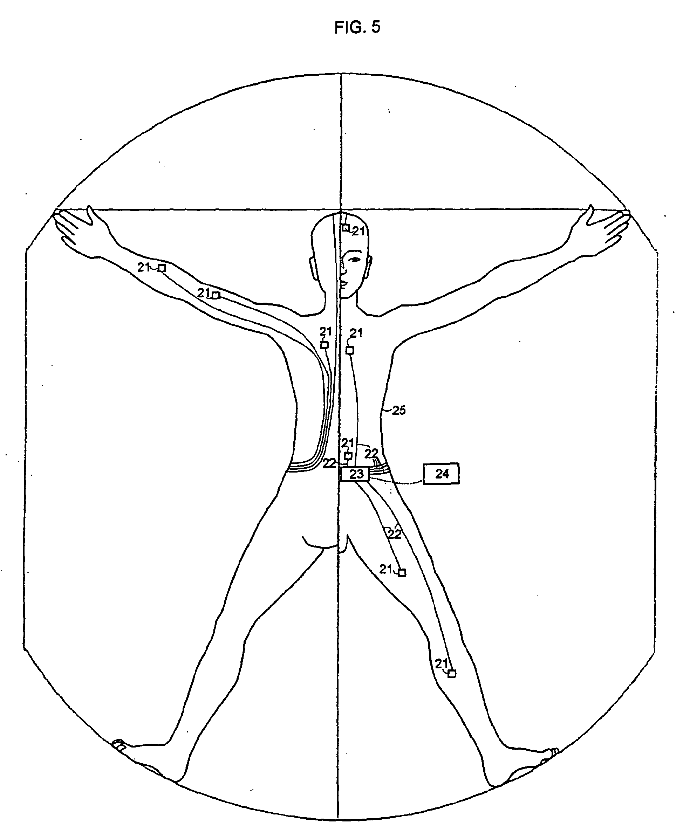

La figure 5 présente la mise en oeuvre de plusieurs dispositifs selon l'invention pour la détermination du climat sous-vestial d'un individu.

La difficulté réside dans le fait que ce climat, par exemple au niveau du tronc 25 d'un individu, est hétérogène et sa caractérisation implique la détermination de valeurs moyennes obtenues par pondération de la mesure en plusieurs sites. Cette pondération doit tenir compte de la surface cutanée explorée. Nous avons donc utilisé une technique similaire à celle employée pour la mesure de la température cutanée moyenne corporelle. Ainsi, des capteurs combinés de température et d'humidité ont été assemblés au sein d'un dispositif 21 selon l'invention de telle façon qu'ils n'entrent en contact ni avec la surface cutanée ni avec les vêtements afin de mesurer les variables thermo-hygrométriques du climat sous-vestial. Le nombre de dispositifs de mesure de la température et de l'humidité selon l'invention utilisés peut varier de 4 à 22 suivant leur localisation sur les différents segments corporels. Au-delà de 22 la précision apportée par les dispositifs supplémentaires n'est pas assez significative pour justifier la présence d'un dispositif supplémentaire. Dans le cas de la figure 5, huit dispositifs 21 de mesure de la température et de l'humidité selon l'invention ont été utilisés: ils ont été placés respectivement, pour ce qui est de la face antérieure de l'individu, au niveau du front, de la poitrine, de l'abdomen, de la cuisse, et de la jambe, et, pour ce qui est de sa face postérieure, au niveau du bras, de l'avant bras et du dos.

Chacun de ces dispositifs est relié par liaison filaire 22 à des moyens d'acquisition et de stockage 23 des signaux provenant de chacun des dispositifs 21, ces moyens comportant en outre soit un module de type Bluetooth^{®} soit une autre liaison numérique, filaire ou non, afin de pouvoir télécharger les données enregistrées par lesdits moyens d'acquisition et de stockage 23 à l'aide de moyens de traitement extérieurs 24 tel, par exemple un ordinateur éventuellement portable. Ces moyens de traitement extérieurs 24 comportent un logiciel spécifique permettant, d'une part, l'acquisition des données enregistrées par lesdits moyens d'acquisition et de stockage 23 et, d'autre part, leur traitement, notamment le calcul de la température moyenne et de la pression partielle moyenne en vapeur d'eau du climat sous-vestial selon la procédure décrite précédemment. Le suivi de ces variables est réalisé quasiment en continu, par exemple à une fréquence de une acquisition par seconde et permet donc la détermination des seuils de température et/ou de pression partielle moyenne de vapeur d'eau pour lesquelles la contrainte physiologique atteint un seuil critique comme ceux mentionnés dans les Normes. Des algorithmes déterminés à partir des expérimentations effectuées permettent de connaître, pour chaque individu, la durée limite d'exposition pour le risque hyperthermique.
Au niveau de chaque dispositif 21, la température et l'humidité relative sont mesurées. L'humidité absolue exprimée en kPa est calculée, dans cet exemple de réalisation, au niveau de chaque site par l'intermédiaire de la formule d'Antoine qui permet la détermination de la pression saturante de vapeur d'eau à la température mesurée au niveau du capteur de température. La pression partielle de vapeur d'eau au niveau du site est ensuite calculée en utilisant l'humidité relative mesurée par le capteur. Ces différentes mesures sont pondérées avec les mêmes coefficients que ceux utilisés pour la détermination de la température cutanée moyenne pour les mêmes sites de mesures. Dans le cas de ce mode de réalisation, la formule de Crawshaw et al. (1975) a été employée. Ainsi, ces calculs permettent la détermination de la température moyenne, de l'humidité relative et absolue moyenne du climat sous-vestial. La détermination de la pression partielle moyenne de vapeur d'eau du climat sous-vestial nous est apparue primordiale car les échanges évaporatoires entre l'ambiance et le climat sous-vestial sont conditionnés par le gradient de pression de vapeur d'eau existant entre le climat sous-vestial et l'environnement. Ainsi, la mesure de la pression partielle moyenne de vapeur d'eau du climat sous-vestial est non seulement le reflet de la contrainte thermique résultant du stockage thermique et donc de la sudation mais aussi le reflet de l'entrave induite par les vêtements à l'évaporation sudorale. Cette évaporation sudorale est le seul moyen efficace de lutte physiologique contre la chaleur. La mesure et le suivi des caractéristiques thermiques du climat sous-vestial et notamment de la pression partielle moyenne de vapeur d'eau permet donc d'anticiper les réponses physiologiques à une contrainte thermique. Ainsi, pour une température moyenne de climat sous-vestial de 37 °C, si la pression partielle moyenne de vapeur d'eau est de 6,25 kPa correspondant à la pression saturante à cette température, la thermolyse évaporatoire ne pourra plus se faire et le sujet ne pourra plus refroidir d'où un risque d'accident d'hyperthermie très élevé. Ce seuil de 6,25 kPa sera atteint bien avant le seuil de température rectale de 39 °C. Cet ensemble de dispositif permet donc, d'une part, de suivre de façon non invasive la contrainte thermique imposée non seulement par l'environnement climatique et la dépense énergétique mais aussi par les équipements vestimentaires et, d'autre part, de prévenir les risques. Cette évaluation des caractéristiques climatiques du climat sous-vestial constitue un premier objectif de l'invention
Un deuxième objectif consiste à utiliser le dispositif pour asservir les systèmes de refroidissement-réchauffement individuels ou collectifs, voire le chauffage ou la climatisation des locaux, aux caractéristiques climatiques du climat sous-vestial telles que décrites précédemment. En effet, la mesure de la température moyenne du climat sous-vestial et de la pression partielle moyenne de vapeur d'eau permet d'ajuster les variables de l'environnement (température, humidité, vent) selon les caractéristiques climatiques du climat sous-vestial. Par exemple, en cas de contrainte thermique chaude importante, dans le cas par exemple du port d'une tenue NRBC, on peut envisager un système de refroidissement asservi au climat sous-vestial afin d'assurer un climat sous-vestial thermiquement confortable donc peu contraignant sur le plan physiologique et efficace sur le plan thermophysiologique. Ceci permet de plus une économie d'énergie et de poids. Il pourrait en être de même avec les systèmes de réchauffement, chauffage-climatisation des locaux ou des véhicules.
Un troisième objectif est la détermination des caractéristiques thermiques des équipements, principalement les résistances thermique et évaporatoire des équipements chez l'homme en activité. Ces mesures nécessitent la mesure de la pression partielle de vapeur d'eau de l'ambiance climatique et la connaissance des pertes évaporatoires en un temps donné pour la détermination de la résistance évaporatoire ; de la dépense énergétique, du stockage ou de la dette thermique et de la température moyenne de surface des équipements pour la détermination de la résistance thermique.

La connaissance de la résistance évaporatoire d'un équipement et du gradient de pression partielle de vapeur d'eau entre le climat sous-vestial et l'environnement permet de quantifier les pertes évaporatoires d'origine sudorale d'un sujet soumis à une charge thermique. Ce dernier point constitue un quatrième objectif d'un dispositif selon l'invention.

La figure 6 présente un exemple d'évolution de la pression partielle moyenne de vapeur d'eau du climat sous-vestial mesuré avec un dispositif de mesure du climat sous-vestial selon la figure 5 pour une personne active évoluant dans une ambiance thermique chaude, à savoir 60°C avec une humidité relative de 80%, cette personne ayant revêtu une combinaison antifeu. On se rend compte que le seuil critique de 6,25 kPa est atteint au bout d'une période très courte de 5 minutes et qu'à partir de cet instant la thermolyse par évaporation sudorale de cette personne ne s'effectue plus correctement et que sa température corporelle interne va donc augmenter très rapidement. Au bout de 20 minutes, sa température corporelle interne moyenne ayant dépassé 39°C, cette personne a dû arrêter son activité sous contrainte pour ne pas courir de risque clinique.

De nombreuses modifications peuvent être apportées aux modes de réalisation décrits précédemment sans sortir du cadre de l'invention.
Ainsi, les deux parties de l'ossature entre lesquelles sont positionnés les capteurs de température et d'humidité peuvent avoir une forme géométrique quelconque tels un cercle, une ellipse, un triangle, un trapèze... et lesdits capteurs peuvent être fixés directement aux dites deux parties. Par ailleurs, dans chacun des exemples décrits, la deuxième partie peut être remplacée par la paroi interne d'un vêtement, d'un sac de couchage, d'un drap, d'une couverture ..., l'une des extrémités de la ou des entretoises étant préférablement fixée à ladite paroi interne.
De plus, on peut associer, à un dispositif selon l'invention, d'autres mesures grâce à des capteurs complémentaires telles que la température rectale, les températures de la peau, les températures de surface des équipements, la température et l'humidité ambiantes, la pression artérielle, la vitesse d'air, la pression barométrique au niveau du climat sous-vestial et au niveau de l'ambiance, la fréquence cardiaque, la saturation artérielle en oxygène (Sp02) ou le pouls et d'autres mesures physiologiques permettant la détermination des risques d'accident hyperthermique ou de déshydratation ... La température ambiante et la pression partielle de vapeur d'eau extérieure de l'environnement climatique, les températures de surface extérieure des équipements sont utiles pour la détermination des résistances évaporatoire et thermique des équipements. La mesure de la pression barométrique du climat sous-vestial est prévue dans le développement du protype afin d'évaluer, d'une part, les surpressions-dépressions voulues (ventilation en surpression par exemple) et, d'autre part, pour une éventuelle correction des pressions partielles de vapeur d'eau du climat sous-vestial.
Avec un dispositif de mesure du climat sous-vestial selon l'invention et certains des capteurs précités, on peut aussi déterminer :
- la résistance évaporatoire d'équipements vestimentaires directement chez l'homme en activité. La connaissance du gradient de pression de vapeur d'eau entre le climat sous-vestial et l'environnement climatique d'une part et les pertes évaporatoires mesurées (différence de poids) du sujet en un temps donné permettent d'en déduire la résistance évaporatoire.
- le niveau des pertes évaporatoires au travers des équipements si la résistance évaporatoire de ceux-ci est connue et différente de 0, correspondant à un vêtement totalement étanche à la vapeur d'eau.
- la résistance thermique d'équipements vestimentaires directement chez l'Homme en activité. Cette évaluation nécessite la connaissance du gradient thermique entre le climat sous-vestial et l'environnement d'une part, la dépense énergétique et le stockage ou la dette thermique, d'autre part.

## Revendications

1. Dispositif de mesure de la température et de l'humidité de l'air situé entre la peau d'un individu et la paroi d'un vêtement ou d'un autre équipement en vis-à-vis de la peau de l'individu, possédant une ossature (1) comportant latéralement selon son épaisseur(e):
- une première partie (4a, 14) constituée par un premier support ajouré apte à être disposé contre la peau de l'individu,
- une seconde partie (4b, 14) constituée par ladite paroi ou par un second support ajouré apte à être disposé contre cette paroi,
- des moyens de liaison ajourés (8, 9, 15, 16) aptes à solidariser la première partie à la seconde partie et constitués soit par une entretoise soit par plusieurs entretoises (8, 9, 15, 16) aptes à permettre une circulation par convexion naturelle de l'air situé entre la peau et ladite paroi au travers du volume délimité par l'ossature,
- au moins un capteur de température (2, 12) et un capteur d'humidité (3, 13) disposés au niveau du plan médian (P1) de l'ossature (1).

2. Dispositif selon la revendication 1, **caractérisé en ce que** la section transversale des moyens de liaison est, dans un plan donné transversal P2, inférieure, à celle, correspondante, du volume de l'ossature (1), leur rapport étant, au maximum égal à 1/2.

3. Dispositif selon l'une quelconque des revendications 1 et 2, **caractérisé en ce que** au moins l'une de la ou des entretoises (8, 9, 15, 16) est constituée par une tige.

4. Dispositif selon l'une quelconque des revendications 1 à 3, **caractérisé en ce que** chacune des dites parties (4a, 4b, 14) est constituée par une plaque ajourée (4a, 4b).

5. Dispositif selon l'une quelconque des revendications 1 à 4, **caractérisé en ce que** chacune des dites parties (4a, 4b, 14) est constituée par un cadre (14) ou par une grille.

6. Dispositif selon l'une quelconque des revendications 1 à 5, **caractérisé en ce que** chacune des dites première et seconde parties (4a, 4b, 14) comporte deux faces principales (5) et **en ce que** l'une et préférablement les deux dimensions principales de ces faces principales (5) est supérieure à l'épaisseur e de l'ossature (1).

7. Dispositif selon l'une quelconque des revendications 1 à 6, **caractérisé en ce qu'**il comporte des moyens (18) de transmission de signaux.

8. Dispositif de mesure du climat sous-vestial d'un individu comportant au moins quatre dispositifs (21) selon l'une quelconque des revendications 1 à 8 aptes à générer des signaux représentatifs de la température et de l'humidité de l'air entourant chacun des capteurs disposés au sein de ces dispositifs.

9. Dispositif selon la revendication 8, **caractérisé en ce qu'**il comporte, en outre, des moyens (23) d'acquisition et de stockage des signaux provenant de chacun des dits au moins quatre dispositifs.

10. Vêtement comportant une face interne destinée à être positionnée en regard de l'individu qui le porte et une face externe opposée, et **caractérisé en ce qu'**il comporte au moins un dispositif (21) selon l'une quelconque des revendications 1 à 9.

11. Vêtement selon la revendication 10, au moins pour le haut du corps et comportant des manches, **caractérisé en ce qu'**il comporte, fixé sur sa paroi interne, au moins un dispositif (21) selon l'une quelconque des revendications 1 à 6 au niveau de l'une des manches, au niveau du torse et au niveau de la taille.

## Claims

1. Device for measuring the temperature and humidity of the air between the skin of an individual and the wall of a garment or other equipment facing the skin of the individual, having a framework (1) including, from side to side according to its thickness (e):
- a first part (4a, 14) consisting of a first skeleton base, and suitable for being arranged against the skin of the individual,
- a second part (4b, 14) consisting of the said wall, or of a second skeleton base, and suitable for being arranged against this wall,
- skeleton linking means (8, 9, 15, 16), which are suitable for connecting the first part positively to the second part, and consisting of either one brace or multiple braces (8, 9, 15, 16), which are suitable for allowing circulation, by natural convection, of the air between the skin and the said wall, through the volume delimited by the framework,
- at least one temperature sensor (2, 12) and one humidity sensor (3, 13) which are arranged at the level of the median plane (PI) of the framework (1).

2. Device according to Claim 1, **characterized in that** the cross-section of the linking means is, in a given transverse plane P2, less than the corresponding one of the volume of the framework (1), their ratio being at maximum equal to 1/2.

3. Device according to any one of Claims 1 and 2, **characterized in that** at least one of the braces (8, 9, 15, 16) consists of a rod.

4. Device according to any one of Claims 1 to 3, **characterized in that** each of the said parts (4a, 4b, 14) consists of a skeleton plate (4a, 4b).

5. Device according to any one of Claims 1 to 4, **characterized in that** each of the said parts (4a, 4b, 14) consists of a frame (14) or grating.

6. Device according to any one of Claims 1 to 5, **characterized in that** each of the said first and second parts (4a, 4b, 14) has two main faces (5), and **in that** one and preferably the two principal dimensions of these main faces (5) is greater than the thickness (e) of the framework (1).

7. Device according to any one of Claims 1 to 6, **characterized in that** it includes means (18) for transmitting signals.

8. Device for measuring the climate under the clothing of an individual, including at least four devices (21) according to any one of Claims 1 to 8, said devices being capable of generating signals representing the temperature and humidity of the air surrounding each of the sensors which are arranged within these devices.

9. Device according to Claim 8, **characterized in that** it also includes means (23) of acquiring and storing signals from each of the at least said four devices.

10. Garment including an internal face which is intended to be positioned facing the individual who is wearing it, and an opposite external face, and **characterized in that** it has at least one device (21) according to any one of Claims 1 to 9.

11. Garment according to Claim 10, at least for the top of the body, and having sleeves, **characterized in that** it has, fixed on its internal wall, at least one device (21) according to any one of Claims 1 to 6, on one of the sleeves, on the torso and on the waist.

## Patentansprüche

1. Vorrichtung zum Messen der Temperatur und der Feuchtigkeit der Luft, die sich zwischen der Haut eines Menschen und der Wand eines Kleidungsstücks oder einer anderen Ausstattung gegenüber der Haut des Menschen befindet, mit einem Gerüst (1), das entsprechend seiner Dicke seitlich folgendes aufweist:
- einen ersten Teil (4a, 14) der aus einem ersten gelochten Träger besteht, der gegen die Haut des Menschen angeordnet werden kann,
- einen zweiten Teil (4b, 14), der aus der Wand oder einem zweiten gelochten Träger besteht, der gegen diese Wand angeordnet werden kann,
- gelochte Verbindungsmittel (8, 9, 15, 16), die den ersten mit dem zweiten Teil fest verbinden können und entweder aus einem Zwischenelement oder mehreren Zwischenelementen (8, 9, 15, 16) bestehen, die dazu geeignet sind, durch natürliche Konvektion ein Zirkulieren der zwischen der Haut und der Wand befindlichen Luft durch das von dem Gerüst begrenzte Volumen hindurch zu ermöglichen,
- mindestens einen Temperatursensor (2, 12) und einen Feuchtigkeitssensor (3, 13), die auf Höhe der mittleren Ebene (P1) des Gerüstes (1) angeordnet sind.

2. Vorrichtung nach Anspruch 1, **dadurch gekennzeichnet, dass** der Querschnitt der Verbindungsmittel in einer gegebenen Querebene P2 kleiner ist als der entsprechende Querschnitt des Volumens des Rahmens (1), wobei deren Verhältnis maximal gleich ½ ist.

3. Vorrichtung nach einem der Ansprüche 1 und 2, **dadurch gekennzeichnet, dass** mindestens ein Zwischenelement aus dem/den Zwischenelement(en) (8, 9, 15, 16) aus einer Stange besteht.

4. Vorrichtung nach einem der Ansprüche 1 bis 3, **dadurch gekennzeichnet, dass** jeder der Teile (4a, 4b, 14) aus einer gelochten Platte (4a, 4b) besteht.

5. Vorrichtung nach einem der Ansprüche 1 bis 4, **dadurch gekennzeichnet, dass** jede der Platten (4a, 4b, 14) aus einem Rahmen (14) oder einem Gitter besteht.

6. Vorrichtung nach einem der Ansprüche 1 bis 5, **dadurch gekennzeichnet, dass** jeder der ersten und zweiten Teile (4a, 4b, 14) zwei Hauptflächen (5) aufweist und dass die eine und vorzugsweise die beiden Hauptabmessungen dieser Hauptflächen (5) größer ist als die Dicke e des Gerüstes (1).

7. Vorrichtung nach einem der Ansprüche 1 bis 6, **dadurch gekennzeichnet, dass** sie Mittel (18) zum Übertragen von Signalen aufweist.

8. Vorrichtung zum Messen des Klimas unter der Kleidung eines Menschen, mit mindestens vier Vorrichtungen (21) nach einem der Ansprüche 1 bis 8, die geeignet sind, repräsentative Signale der Temperatur und der Feuchtigkeit der Luft zu erzeugen und die jeweils Sensoren umgeben, die innerhalb dieser Vorrichtungen angeordnet sind.

9. Vorrichtung nach Anspruch 8, **dadurch gekennzeichnet, dass** sie zudem Mittel (23) zum Erfassen und Speichern der Signale aufweist, die von jeder der mindestens vier Vorrichtungen stammen.

10. Kleidung mit einer Innenseite, die dazu bestimmt ist, gegenüber dem Menschen positioniert zu werden, der sie trägt, und einer entgegengesetzten Außenseite, **dadurch gekennzeichnet, dass** sie mindestens eine Vorrichtung (21) nach einem der Ansprüche 1 bis 9 aufweist.

11. Kleidung nach Anspruch 10, mindestens für den oberen Teil des Körpers und mit Ärmeln, **dadurch gekennzeichnet, dass** sie auf Höhe eines der Ärmel, auf Höhe des Rumpfes und auf Höhe der Taille mindestens eine an ihrer Innenseite befestigten Vorrichtung (21) nach einem der Ansprüche 1 bis 6 aufweist.
